# EUROPEAN PATENT APPLICATION

(11) **EP 2 258 719 A1**
(43) Date of publication of application: **08.12.2010**
(21) Application number: 09160679.8
(22) Date of filing: 19.05.2009
(51) Int. Cl.: C07K 14/705, C12N 15/62, A61K 38/17, A61P 35/00

(54) **Multiple target T cell receptor**

(71) Applicant: Max-Delbrück-Centrum für Molekulare Medizin (MDC), 13125 Berlin (DE)
(72) Inventor: Blankenstein, Thomas, Prof. Dr., 13467 Berlin (DE); Charo, Jehad, Dr., 10115 Berlin (DE); Uckert, Wolfgang, Prof. Dr., 13125 Berlin (DE); Kieback, Elisa, 10405 Berlin (DE); Perez, Cynthia, 10365 Berlin (DE)
(74) Representative: Sandmann, Wolfgang

(57) **Abstract**

The present invention is directed to a functional T cell receptor (TCR) fusion protein (TFP) recognizing and binding to at least one MHC-presented epitope, and containing at least one amino acid sequence recognizing and binding an antigen. The present invention is further directed to an isolated nucleic acid molecule encoding the same, a T cell expressing said TFP, and a pharmaceutical composition for use in the treatment of diseases involving malignant cells expressing said tumor-associated antigen.

SEQUENCE LISTING

<110>  Max-Delbrück-Centrum für Molekulare Medizin (MDC)

<120>  Multiple target T cell receptor

<130>  P26813

<160>  4

<170>  PatentIn version 3.3

<210>  1
<211>  6
<212>  PRT
<213>  artificial

<220>
<223>  peptide MARS

<400>  1

Met Ala Arg Ser Gly Leu
1               5


<210>  2
<211>  9
<212>  PRT
<213>  artificial

<220>
<223>  peptide RGD4C

<400>  2

Cys Asp Cys Arg Gly Asp Cys Phe Cys
1               5


<210>  3
<211>  10
<212>  PRT
<213>  artificial

<220>
<223>  myc-tag

<400>  3

Glu Gln Lys Leu Ile Ser Glu Glu Asp Leu
1               5                   10


<210>  4
<211>  20
<212>  PRT
<213>  artificial

<220>
<223>  2xmyc-tag

<400>  4

Glu Gln Lys Leu Ile Ser Glu Glu Asp Leu Glu Gln Lys Leu Ile Ser
1               5                   10                  15

Glu Glu Asp Leu
            20

## Description

### FIELD OF THE INVENTION

The present invention is directed to a functional T cell receptor (TCR) fusion protein (TFP) recognizing and binding to at least one MHC-presented epitope, and containing at least one amino acid sequence recognizing and binding an antigen. The present invention is further directed to an isolated nucleic acid molecule encoding the same, a T cell expressing said TFP, and a pharmaceutical composition for use in the treatment of diseases involving malignant cells expressing said tumor-associated antigen.

### BACKGROUND OF THE INVENTION

TCR's are members of the immunoglobulin superfamily and usually consist of two subunits, namely the α- and β-subunits. These possess one N-terminal immunoglobulin (Ig)-variable (V) domain, one Ig-constant (C) domain, a transmembrane/cell membrane-spanning region, and a short cytoplasmic tail at the C-terminal end. The variable domains of both the TCR α-chain and β-chain have three hypervariable or complementarity determining regions (CDRs), whereas the variable region of the β-chain has an additional area of hypervariability (HV4) that does not normally contact the MHC-peptide complex and therefore is not considered a CDR.

CDR3 is the main CDR responsible for recognizing processed antigen, although CDR1 of the alpha chain has also been shown to interact with the N-terminal part of the antigenic peptide, whereas CDR1 of the β-chain interacts with the C-terminal part of the peptide. CDR2 is thought to recognize the MHC. HV4 of the β-chain is not thought to participate in MHC-peptide complex recognition, but has been shown to interact with superantigens. The constant domain of the TCR domain consists of short connecting sequences in which a cysteine residue forms disulfide bonds, which forms a link between the two chains.

The affinity of TCR's for a specific antigen makes them valuable for several therapeutic approaches. For example, cancer patients, such as melanoma patients, can be effectively treated by using adoptive immunotherapy. That is to say, significant tumor regression can occur following adoptive transfer of T cells with anti-tumor specificity. However, patient-derived T cells may have sub-optimal activity. Therefore, there is current interest in using pre-characterized TCR genes to create designer lymphocytes for adoptive cell therapies.

The first clinical trials using adoptive transfer of TCR-transgenic T cells showed some clinical efficacy. However, the problem appears that therapies might fail due to so called antigen loss variants developed by cancer cells. Antigen- or MHC-loss variants are frequent mechanisms used by cancer cells that lead to their escape from detection and elimination by T cells (Seliger 2005). Whether it is due to active immune selection, as demonstrated by immunotherapy studies (Restifo et al. 1996; Khong and Restifo 2002) or due to genomic instability, required to maintain tumor outgrowth and the acquisition of its fully malignant phenotype, these escape mechanisms are very heterogeneous. They include mutations that lead to decreased or no MHC expression such as mutations in the MHC molecule heavy chains encoding genes or in those encoding for molecules involved in antigen processing and presentation including transporter associated with antigen presentation (TAP) and the light chain of MHC-I molecule; β2-microglobulin (Khong and Restifo 2002; Seliger 2005).

Altered level of expression of MHC can also be due to an effect mediated by suppressive cytokines such as interleukin 10. Recognition of tumor associated antigens can also be lost due to mutations or decreased expression level of these antigens (Khong and Restifo 2002). This was shown for melanoma antigens as well as for carcinomas and in an experimental mouse model and was associated with immune ignorance (Spiotto et al. 2002; Matsui et al. 2003; Spiotto and Schreiber 2005).

Another suggested mechanism for tumor escape from immune recognition is based on immunodominance (Schreiber et al. 2002). This mechanism favours sequential escape and selection of cancer cells expressing subdominant or cryptic antigen loss variants that can not be recognised by the mislead immune system.

The development of antigen loss variants represents a serious challenge for any successful immunotherapy approach, which is often based on targeting a single antigen or even an epitope (Leen et al. 2007). Yet, each single cancer cell expresses several antigens and 6-12 different MHC class I and II alleles (Schreiber et al. 2002; Janeway et al. 2004). Studies of tumor-specific and -associated antigens have led to the isolation of many potential immunotherapy targets (Wang et al. 2006). With this myriad of targets, the earnest is on the ability of using these targets to kill tumor cells via the utilisation of their counter receptors.

Therapeutic interventions based on using monoclonal antibodies or the transfer of T cells or natural killer (NK) cells engineered with T cell receptor (TCR) or chimeric receptor genes represent a promising advance in immunotherapy (Leen et al. 2007; Ljunggren and Malmberg 2007). Several tumor-recognising antibodies and TCRs were recently isolated and used in experimental studies and in clinical trials and the list is continuously growing (Waldmann 2006; McKee et al. 2007). Furthermore, some antibodies have already passed the scrutiny of regulatory bodies and are being currently used as a standard therapy (Waldmann 2006). These include antibodies targeting angiogenesis, which is an essential process for solid tumor formation and survival (Folkman 2007).

Alternatively, targeting tumors with short peptide sequences derived from phage display or synthetic peptide library represents a promising approach in biotherapy (Arap et al. 1998; Scott 2001). Among others, peptides specific for breast, prostate and colon carcinomas as well as those specific for neo-vasculatures were successfully isolated (Samoylova et al. 2006).

Both experimental data and theoretical considerations suggest that the most effective approach of treating cancer would be through a vigorous immune response that can prevent the development of loss variants and enables the eradication of a large established tumor (Spiotto et al. 2002; Matsui et al. 2003; Spiotto and Schreiber 2005). This was most clearly established by the studies reported from H. Schreiber's lab, wherein successful T-cell immunotherapy was shown to operate at established tumors only when this tumor expressed a high level of the antigen, in a process that required recognition of the tumor stroma. Conversely, tumors established from the very same cell line but have a low level of the antigen expressed escaped the immune destruction and allowed the development of antigen-loss variant.

Therefore, new therapies are needed in order to address the above mentioned drawbacks of known therapeutic approaches. These new therapies should provide a vigorous immune response that can prevent the development of loss variants and should enable the eradication of a large established tumor.

### SUMMARY OF THE INVENTION

Therefore, it is an object of the present invention to provide a new therapeutic approach which allows an improved therapy of several diseases, such as tumor related diseases, autoimmune, hereditary, or infectious diseases, immunodeficiency, allergy and which might find application in transplantation.

It is a further object of the present invention to provide a functional TCR fusion protein, which shows high affinity against tumor-associated antigens and MHC-presented epitopes. It is a still further object of the invention to provide pharmaceutical compositions for use in adoptive cell therapy which allow an effective treatment of the diseases and conditions outlined above. In particular, it is an object of the invention to provide a functional TFP, which can overcome the problems associated with tumor escape variants due to antigen loss or major histocompatibility complex protein loss and which may target neo-vasculature-specific antigens at the same time.

These objects are achieved by the subject-matter of the independent claims. Preferred embodiments are indicated in the dependent claims.

Herein, a new type of therapy is disclosed that will target simultaneously at least two different antigens and may further target angiogenesis as well. This is exemplified by targeted antigens such as HER-2, a cell surface molecule that is presented independently of MHC restriction and the MHC-presented epitopes gp100₂₀₉ and gp33 derived from the melanoma associated antigen gp100 or the model tumor antigen gp1 from the lymphocytic choriomeningitis virus, respectively. Angiogenesis may, for example, be targeted through the integrins αvβ3 and its homologue αvβ5 expressed on tumor neo-vasculatures (Arap et al. 1998; Scott 2001).

The inventors surprisingly could show that a TCR fusion protein having at least two functionalities, i.e. one for a MHC-presented epitope, and one for an additional antigen, can be generated without affecting the original specificity for the MHC-complex.

Therefore, the TCR of the invention and the therapeutic approach disclosed herein enables multiple targeting of tumors via one genetic modification using one TCR. This is achieved by providing a TCR fusion protein which allows peptide guided and TCR guided attack of cells, in particular tumor cells, at the same time. Thus, the TCR fusion protein as disclosed herein will allow a remarkable improvement of therapies relying on adoptive T cell transfer.

### DETAILED DESCRIPTION OF THE INVENTION

According to a first aspect, the present invention provides a functional TCR α and/or β chain fusion protein recognizing and binding to at least one MHC-presented epitope, and containing at least one amino acid (or peptide) sequence recognizing and binding an antigen. A TCR of the present invention may comprise only one of the α-chain or β-chain sequences as defined herein (in combination with a further α-chain or β-chain, respectively) or may comprise both chains.

In the context of the present invention, a "functional" T-cell receptor (TCR) α- and/or β-chain fusion protein shall mean an α- and/or β-chain fusion protein that, although the chain includes a (foreign) amino acid sequence or peptide, maintains at least substantial biological activity in the fusion protein. In the case of the α- and/or β-chain of a TCR, this shall mean that both chains remain able to form a T-cell receptor (either with a non-modified α- and/or β-chain or with another fusion protein α- and/or β-chain) which exerts its biological function, in particular binding to the specific peptide-MHC complex of said TCR, and/or functional signal transduction upon peptide activation.

As outlined above, the present functional TCR fusion protein has two basic functions: one is directed against an MHC-presented epitope, the other is a peptide-based or peptide-guided function in order to target an antigen. The peptide-guided function can in principle be achieved by introducing peptide sequences into the TCR and by targeting tumors with these peptide sequences. These peptides may be derived from phage display or synthetic peptide library (see supra, Arap et al. 1998; Scott 2001). Among others, peptides specific for breast, prostate and colon carcinomas as well as those specific for neo-vasculatures were already successfully isolated and may be used in the present invention (Samoylova et al. 2006).

In a preferred embodiment, the epitope is a tumor cell epitope and the antigen is a tumor cell antigen. Such a tumor cell epitope may be derived from a wide variety of tumor antigens such as antigens from tumors resulting from mutations, shared tumor specific antigens, differentiation antigens, and antigens overexpressed in tumors. Those antigens, for example may be derived from alpha-actinin-4, ARTC1, BCR-ABL fusion protein (b3a2), B-RAF, CASP-5, CASP-8, beta-catenin, Cdc27, CDK4, CDKN2A, Cox-1, dek- can fusion protein, EFTUD2, Elongation factor 2, ETV6-AML1 fusion protein, FLT3-ITD, FN1, GPNMB, LDLR-fucosyltransferaseAS fusion protein, HLA-A2^{d}, HLA-A11^{d}, hsp70-2, KIAA0205, MART2, ME1, MUM-1^{f}, MUM-2, MUM-3, neo-PAP, Myosin class I, NFYC, OGT, OS-9, p53, pml-RARalpha fusion protein, PRDX5, PTPRK, K-ras, N-ras, RBAF600, SIRT2, SNRPD1, SYT-SSX1- or -SSX2 fusion protein, TGF-betaRII, triosephosphate isomerase, BAGE-1, GAGE-1, 2, 8, Gage 3, 4, 5, 6, 7, GnTV^{f}, HERV-K-MEL, KK-LC-1, KM-HN-1, LAGE-1, MAGE-A1, MAGE-A2, MAGE-A3, MAGE-A4, MAGE-A6, MAGE-A9, MAGE-A10, MAGE-A12, MAGE-C2, mucin^{k}, NA-88, NY-ESO-1/LAGE-2, SAGE, Sp17, SSX-2, SSX-4, TAG-1, TAG-2, TRAG-3, TRP2-INT2^{g}, XAGE-1b, CEA, gp100/Pme117, Kallikrein 4, mammaglobin-A, Melan-A/MART-1, NY-BR-1, OA1, PSA, RAB38/NY-MEL-1, TRP-1/gp75, TRP-2, tyrosinase, adipophilin, AIM-2, ALDH1A1, BCLX (L), BING-4, CPSF, cyclin D1, DKK1, ENAH (hMena), EP-CAM, EphA3, EZH2, FGF5, G250/MN/CAIX, HER-2/neu, IL13Ralpha2, intestinal carboxyl esterase, alpha fetoprotein, M-CSFT, MCSP, mdm-2, MMP-2, MUC1, p53, PBF, PRAME, PSMA, RAGE-1, RGS5, RNF43, RU2AS, secernin 1, SOX10, STEAP1, surviving, Telomerase, VEGF, or WT1, just to name a few.

Further information in this regard my be derived from the data described in Nucleic Acids Research, 2006, Vol. 34, Database issue D607-D612; in tables 3a) and b) of Folkman et al., Angiogenesis: an organizing principle for drug discovery?, Nature Reviews Drug Discovery, Vol. 6, April 2007; and in table 1 of Langenkamp et al., Microvascular endothelial cell heterogeneity: general concepts and pharmacological consequences for anti-angiogenic therapy of cancer, Cell Tissue Res (2009) 335:205-222. The aforementioned publications are incorporated herein by reference.

In a further embodiment, peptide sequences introduced into the TCR are providing a peptide-guided recognition and binding of an antigen which is a cell-surface antigen, a further MHC-presented epitope or an extracellular antigen, preferably a neo-vasculature specific antigen.

The cell surface antigen or the further MHC-presented epitope preferably is derived from HER2, CEA, PSMA, CD20 or one or more of the above mentioned antigens. The epitope is preferably selected from gp100 or gp1, or as well from one or more of the above mentioned antigens.

In a preferred embodiment, the amino acids (or peptides) recognizing and binding to an antigen are targeting a neo-vasculature specific antigen. Those antigens are valuable and promising targets in the attack on tumor cells. See, in this connection the above mentioned publications of Folkman et al., Angiogenesis: an organizing principle for drug discovery?, Nature Reviews Drug Discovery, Vol. 6, April 2007, and Langenkamp et al., Microvascular endothelial cell heterogeneity: general concepts and pharmacological consequences for anti-angiogenic therapy of cancer, Cell Tissue Res (2009) 335:205-222.

Preferably, the neo-vasculature specific antigen is integrin αvβ3 or αvβ5. For a further selection of antigens, see also table 3a) and b) of Folkman et al. and table 1 of Langenkamp et al.

According to a further preferred embodiment, the TCR fusion protein of the invention is **characterized in that**
i) the one or more antigen binding amino acid (peptide) sequences are added to the N-terminus of the α and/or β chain,
ii) the one or more antigen binding amino acid sequences are inserted into a constant or a variable region of said α and/or β chain, and/or
iii) the one or more antigen binding amino acid sequences are replacing a number of amino acids in a constant or a variable region of said α and/or β chain.

This selection of insertion sites of the amino acid sequence guarantees a proper function of the overall TCR, i.e. its biological function, in particular binding to the specific peptide-MHC complex of said TCR, and/or functional signal transduction upon peptide activation.

Preferably, said fusion protein comprises the one or more antigen binding amino acid sequences either spaced apart or directly in tandem, or the TCR is expressed as a single chain or soluble receptor.

In a further embodiment, the TCR α and/or β chain fusion protein of the invention may further comprise at least one epitope-tag, wherein said epitope-tag is selected from
i) an epitope-tag added to the N-terminus of the α and/or β chain,
ii) an epitope-tag inserted into a constant or variable region of said α and/or β chain, and
iii) an epitope-tag replacing a number of amino acids in a constant or variable region of said α and/or β chain.

Preferred is a functional T-cell receptor (TCR) α- and/or β-chain fusion protein according to the present invention, wherein said epitope-tag is selected from, but not limited to, CD20 or HER2/neu tags, or other conventional tags such as a myc-tag, FLAG-tag, T7-tag, HA (hemagglutinin)-tag, His-tag, S-tag, GST-tag, or GFP-tag. myc, T7, GST, GFP tags are epitopes derived from existing molecules. In contrast, FLAG is a synthetic epitope tag designed for high antigenicity (see, e.g., U.S. Pat. Nos. 4,703,004 and 4,851,341). The myc- tag can preferably be used because high quality reagents are available to be used for its detection. Epitope tags can of course have one or more additional functions, beyond recognition by an antibody. The sequences of these tags are described in the literature and well known to the person of skill in art.

In the functional T-cell receptor (TCR) α- and/or β-chain fusion protein according to the present invention, said fusion protein may be for example selected from two myc-tag sequences that are attached to the N-terminus of an α-TCR-chain and/or 10 amino acids of a protruding loop region in the β-chain constant domain being exchanged for the sequence of two myc-tags.

In an embodiment of the present invention, the inventors inserted an amino acid sequence that corresponds to a part of the myc protein (myc-tag) at several reasonable sites into the structure of a T cell receptor and transduced this modified receptor into T cells. By introducing a tag into the TCR structure, it is possible to deplete the modified cells by administering the tag-specific antibody to the patient.

Those functional TCR fusion proteins may be used in a method for selecting a host cell population expressing a fusion protein selected from the group consisting of a fusion protein comprising a) at least one epitope-providing amino acid sequence (epitope-tag), and b) the amino acid sequence of an α- and/or β-chain of a TCR as defined above, wherein said epitope-tag is selected from an epitope-tag added to the N-terminus of said α- and/or β-chain or added into the α- and/or β-chain sequence, but outside the CDR3 region, an epitope-tag inserted into a constant region of said α- and/or β-chain, and an epitope-tag replacing a number of amino acids in a constant region of said α- and/or β-chain; and a TCR comprising at least one fusion protein as above on the surface of the host cell; comprising contacting host cells in a sample with a binding agent that immunologically binds to the epitope-tag, and selection of said host cells based on said binding.

In the TCR fusion protein, the amino acid sequence recognizing and binding an antigen has preferably a length of between 5-20 amino acids, more preferably between 6 and 12 amino acids. The amino acid sequence recognizing and binding an antigen is preferably selected from SEQ ID NO: 1 and/or 2 corresponding to MARSGL and CDCRGDCFC.

In a second aspect, the present invention provides an isolated nucleic acid coding for the TCR fusion protein as defined herein.

The term "isolated nucleic acid" as used herein with reference to nucleic acids refers to a naturally-occurring nucleic acid that is not immediately contiguous with both of the sequences with which it is immediately contiguous (one on the 5' end and one on the 3' end) in the naturally-occurring genome of the cell from which it is derived. For example, a nucleic acid can be, without limitation, a recombinant DNA molecule of any length, provided one of the nucleic acid sequences normally found immediately flanking that recombinant DNA molecule in a naturally-occurring genome is removed or absent. Thus, a nucleic acid includes, without limitation, a recombinant DNA that exists as a separate molecule (e. g., a cDNA or a genomic DNA fragment produced by PCR or restriction endonuclease treatment) independent of other sequences as well as recombinant DNA that is incorporated into a vector, an autonomously replicating plasmid, a virus (e. g., a retrovirus, or adenovirus). In addition, an isolated nucleic acid can include a recombinant DNA molecule that is part of a hybrid or fusion nucleic acid sequence.

Furthermore, the term "isolated nucleic acid" as used herein also includes artificially produced DNA or RNA sequences, such as those sequences generated by DNA synthesis based *on in silico* information.

The nucleic acids of the invention can comprise natural nucleotides, modified nucleotides, analogues of nucleotides, or mixtures of the foregoing as long as they are capable of causing the expression of a polypeptide *in vitro,* and preferably, in a T cell. The nucleic acids of the invention are preferably RNA, and more preferably DNA, but may also take the form of PNA, CNA, mRNA or mixtures thereof.

In a third aspect, the invention is directed to a vector, preferably a plasmid, shuttle vector, phagemide, cosmid, expression vector, retroviral vector, adenoviral vector or particle and/or vector to be used in gene therapy, which comprises the above defined nucleic acid molecule.

In the context of the present invention, a "vector" shall mean a nucleic acid molecule as introduced into a host cell, thereby producing a transformed host cell. A vector may include nucleic acid sequences that permit it to replicate in a host cell, such as an origin of replication. A vector may also include one or more selectable marker genes and other genetic elements known to those of ordinary skill in the art. A vector preferably is an expression vector that includes a nucleic acid according to the present invention operably linked to sequences allowing for the expression of said nucleic acid.

A fourth aspect provides a host cell transfected with a vector or a nucleic acid as defined above , or which has been infected or transduced with a particle as described above.

The host cell preferably is a T-cell, a natural killer cell, a monocyte, a natural killer T-cell, a monocyte- precursor cell, a hematopoietic stem cell or a non-pluripotent stem cell. More preferably, it is a peripheral blood lymphocyte (PBL) which has been transfected or transduced with the above vector. The step of cloning the T cell receptor (TCR) of the isolated T cells and/or expressing the TCR transgenes in PBMC can be done according to established methods.

In a still further preferred embodiment, the host cell expresses a fusion protein as disclosed herein on its surface.

A fifth aspect of the present invention provides a method for producing a fusion protein according to the invention, comprising a chemical synthesis or genetic engineering of said peptide. Those methods for chemically synthesizing proteins and peptides are described in the literature, and well known to the person of skill.

Peptides (at least those containing peptide linkages between amino acid residues) may be synthesized by the Fmoc-polyamide mode of solid-phase peptide synthesis as disclosed by Lu et al (1981) J. Org. Chem. 46, 3433-3436, and references therein. Temporary N-amino group protection is afforded by the 9-fluorenylmethyloxycarbonyl (Fmoc) group. Repetitive cleavage of this highly base-labile protecting group is achieved by using 20 % piperidine in N, N-dimethylformamide. Side-chain functionalities may be protected as their butyl ethers (in the case of serine threonine and tyrosine), butyl esters (in the case of glutamic acid and aspartic acid), butyloxycarbonyl derivative (in the case of lysine and histidine), trityl derivative (in the case of cysteine) and 4-methoxy-2,3,6-trimethylbenzenesulphonyl derivative (in the case of arginine). Where glutamine or asparagine are C-terminal residues, use is made of the 4,4'-dimethoxybenzhydryl group for protection of the side chain amido functionalities. The solid-phase support is based on a polydimethyl-acrylamide polymer constituted from the three monomers dimethylacrylamide (backbone-monomer), bisacryloylethylene diamine (cross linker) and acryloylsarcosine methyl ester (functionalizing agent). The peptide-to-resin cleavable linked agent used is the acid-labile 4-hydroxymethyl- phenoxyacetic acid derivative. All amino acid derivatives are added as their preformed symmetrical anhydride derivatives with the exception of asparagine and glutamine, which are added using a reversed N, N-dicyclohexyl-carbodiimide/lhydroxybenzotriazole mediated coupling procedure. All coupling and deprotection reactions are monitored using ninhydrin, trinitrobenzene sulphonic acid or isotin test procedures.

Upon completion of synthesis, peptides are cleaved from the resin support with concomitant removal of side-chain protecting groups by treatment with 95% trifluoroacetic acid containing a 50 % scavenger mix. Scavengers commonly used are ethanedithiol, phenol, anisole and water, the exact choice depending on the constituent amino acids of the peptide being synthesized. Trifluoroacetic acid is removed by evaporation in vacuo, with subsequent trituration with diethyl ether affording the crude peptide. Any scavengers present are removed by a simple extraction procedure which on lyophilization of the aqueous phase affords the crude peptide free of scavengers. Reagents for peptide synthesis are generally available from Calbiochem- Novabiochem (UK) Ltd, Nottingham NG7 2QJ, UK.

Purification may be effected by any one, or a combination of, techniques such as size exclusion chromatography, ion-exchange chromatography and (usually) reverse-phase high performance liquid chromatography.

Analysis of peptides may be carried out using thin layer chromatography, reverse-phase high performance liquid chromatography, amino-acid analysis after acid hydrolysis and by fast atom bombardment (FAB) mass spectrometric analysis, as well as MALDI and ESI-Q-TOF mass spectrometric analysis.

Furthermore, the fusion protein may be prepared by using genetic engineering: such a method, as an example, may comprise expressing a nucleic acid molecule encoding a fusion protein according to the present invention in a host cell of the invention, and purifying said fusion protein or said TCR from said host cell.

One skilled in the art will understand that there are myriad ways to express a recombinant protein such that it can subsequently be purified. In general, an expression vector carrying the nucleic acid sequence that encodes the desired fusion protein will be transformed into a microorganism for expression. Such microorganisms can be prokaryotic or eukaryotic. A eukaryotic expression system will be preferred where the protein of interest requires eukaryote-specific post-translational modifications such as glycosylation. Also, protein can be expressed using a viral (e.g., vaccinia) based expression system.

Protein can also be expressed in animal cell tissue culture, and such a system will be appropriate where animal-specific protein modifications are desirable or required in the recombinant protein. Such expression is particularly appropriate where native assembly and export of the inventive fusion protein is desirable, since the activity of TCRs is influenced by native dimerization (folding and assembly) and presentation on the cell.

In a sixth aspect, the present invention provides a pharmaceutical composition which comprises a fusion protein, a nucleic acid molecule, a vector according or a host cell as defined hereinabove, together with a pharmaceutically acceptable carrier or excipient.

Those active components of the present invention are preferably used in such a pharmaceutical composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutically acceptable" defines a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve synergistic effects or to minimize adverse or unwanted effects.

Techniques for the formulation or preparation and application/medication of active components of the present invention are published in "Remington's Pharmaceutical Sciences", Mack Publishing Co., Easton, PA, latest edition. An appropriate application is a parenteral application, for example intramuscular, subcutaneous, intramedular injections as well as intrathecal, direct intraventricular, intravenous, intranodal, intraperitoneal or intratumoral injections. The intravenous injection is the preferred treatment of a patient. According to a preferred embodiment, the pharmaceutical composition is an infusion or an injection.

An injectable composition is a pharmaceutically acceptable fluid composition comprising at least one active ingredient, e.g., an expanded T-cell population (for example autologous or allogenic to the patient to be treated) expressing a TCR. The active ingredient is usually dissolved or suspended in a physiologically acceptable carrier, and the composition can additionally comprise minor amounts of one or more non-toxic auxiliary substances, such as emulsifying agents, preservatives, and pH buffering agents and the like. Such injectable compositions that are useful for use with the fusion proteins of this disclosure are conventional; appropriate formulations are well known to those of ordinary skill in the art.

According to a further aspect, the present invention is directed to a method of treating a patient in need of adoptive cell therapy, said method comprising administering to said patient a pharmaceutical composition as defined above. The disease to be treated preferably is a tumor, autoimmune, hereditary, or infectious disease, immunodeficiency, allergy and/or the composition will be designed for use in transplantation.

The present invention now will be illustrated by the enclosed Figures and the Examples. The following examples further illustrate the invention but, of course, should not be construed as limiting its scope.

### DESCRIPTION OF THE FIGURES

Figure 1: Jurkat MA T cells untransduced or transduced with the wilde-type (WT) or with the myc-taged gp100 (DAN) TCRs were unstimulated or stimulated with a CD3 (OKT-3) antibody as a positive control, or with an anti myc-tag (9E10) antibody. Response was measured using the luciferin conversion assay and measured in relative light unit.
Figure 2: Schematic representation of the TCR (left) and the TFPhv (right) with the HER-2 specific peptide (MARSGL) and the neo-vasculature integrins αvβ3 and αvβ5 specific peptide (RGD4C) are depicted at the N-termini of the TCR molecule.

### EXAMPLES

The P14 TCR, which recognises the gp33 epitope from the lymphocytic choriomeningitis virus, was studied for identifying neutral sites for the introduction of peptide sequences. Similarly, the gp100 TCR, which recognises the human melanoma antigen derived epitope gp100₂₀₉ was studied for identifying such sites. This was achieved by introducing a myc-tag sequence (table 1) into 11 different sites of the TCR molecule, five of which were used in both TCRs resulting in a total number of 16 different TFPs. T cells transduced with any of these 16 constructs expressed the TFP at a level similar to that of the wild type P14 or gp100 TCRs and maintained their specificity to the corresponding tetramer and to stimulation by the specific peptide-pulsed target.

### Testing TFP for the ability of responding to de novo stimulation:

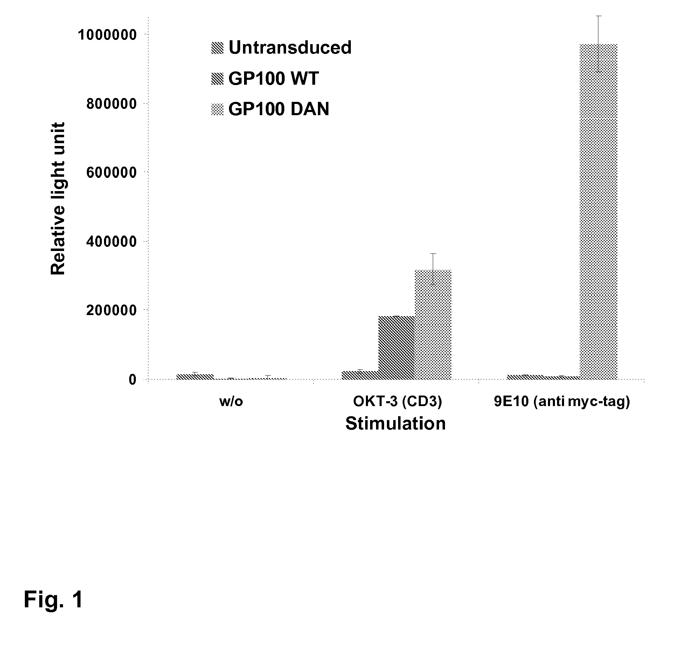

The inventors have recently used CD3 or myc-tag specific antibodies to investigate the possibility of stimulating the transduced T cells. They found that 9 of the 16 TFPs located in 3 different positions of the TFPs can be triggered by the myc-tag specific antibody to produce similar amount of IL-2 to that produced through the control stimulation by the CD3 antibody (Fig. 1). These data indicate that a targeting-peptide introduced to the TCR can be used to trigger the TCR similarly to the triggering of this molecule by the corresponding MHC-peptide complex and provides 3 potential sites for the introduction of targeting peptides.

### Molecular cloning of TFP encompassing TCR with three specificities:

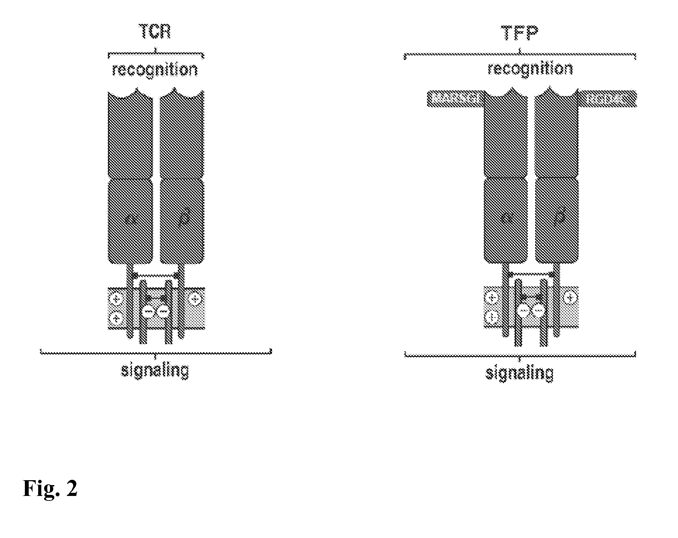

Peptide sequences specific for the tumor-associated antigen HER-2 and/or tumor vasculature (based on the RGD4C sequence) indicated in table 1 are cloned into the identified optimal sites of the TCR sequences. This is achieved by introducing the corresponding nucleotide sequences into the TCR chains using overlapping PCR primers. Unique restriction sites with silent substitutions are introduced to enable a potential further cloning of alternative targeting peptides. Constructs identities are confirmed by restriction mapping and sequencing. The resulting TFPs recognise HER-2, neovasculature and gp33-expressing H2Kb target cells or gp100₂₀₉ HLA-A2-expressing target cells. Three different types of TFPs with specificities to TCR epitope and HER-2 (TFPh), TCR epitope and angiogenesis (TFPv) or TCR epitope, HER-2 and angiogenesis (TFPhv) are constructed (Fig.2).

### Establishing a tumor cell line with two different model antigens with the expression level of one being controllable:

A tumor cell line is constructed in which the level of HER-2 will be constant, while the level of gp33 is controlled by an inducible cre recombination system based on the MC57G-gp33 cell line provided by Dr. H. Schreiber, Chicago. In this system the gp33 eptitope is expressed at low but detectable levels due to inhibition by a floxable sequence containing the HLA-A2 and puromycin. Following cre recombination, which is induced by tamoxifen administration, this inhibitory sequence is floxed, which enables a higher expression level for the gp33 epitope. The uninduced tumor cell line is designated MC57G-gp33_{low}, and the induced cell line is designated MC57G-gp33_{high}. In this model a control cell line MC57G-neo, which expresses the inducible cre construct is used as a gp33 negative cell line.

HER-2 is introduced to the uninduced MC57G-gp33 or to MC57G-neo by retroviral transduction using the MIG-R1 HER-2 construct (provided by Dr. Helga Bernhard, GSF, Munich). The resulting tumor cell lines are designated MC57G-neo-HER-2, MC57G-gp33_{low}-HER-2, and MC57G-gp33_{high}-HER-2.

### T cell transduction

Human T cells are derived from peripheral blood lymphocytes, while mouse T cells are derived from splenocytes of OT-I-Rag-/- TCR transgenic mice.

T cells are transduced with the different TFPs by spinocculation. Cryopreserved peripheral blood mononuclear cells are collected from healthy donor blood donation by centrifugation on Ficoll-Hypaque gradients. Peripheral blood mononuclear cells are stimulated with 10 ng/mL CD3 (ORTHOCLONE OKT3) and 600 IU IL-2/mL. The retroviral production is done in 293T cells that are cotransfected with the TFP encoding plasmid or GFP as a mock control and the pCL-10A1 plasmid encoding gag, pol and env using lipofection. Virus supernatant is collected and used to transduce primary activated human T cells three days after the activation. Enrichment of mouse T cells for retroviral transduction is achieved by isolating splenocytes from OT-I mice, followed by red blood cell lyses by ammonium chloride solution and stimulation with the OT-I epitope (SIINFEKL), anti CD3 and anti CD28 antibodies. The ecotropic virus supernatant is produced by transiently transfecting plasmid-DNA encoding the appropriate TFP or GFP as a mock control using lipofection into the ecotropic packaging cell line Plat-E. T cells are transduced on two subsequent days and the transduction efficacy will be analysed by FACS.

### Measuring TFP expression and binding capacity to three independent antigens

TFPs expression is confirmed using specific antibodies, tetramers and by using recombinant proteins followed by specific antibody staining. This is achieved using commercially available recombinant HER-2 or αvβ3 (which binds to the RGD4C peptide) proteins and antibodies specific to these proteins. Cells expressing the TFPs or control TCRs are incubated with the recombinant protein for 30 minutes after which the residual non-binding protein is washed away. Fluorochrome-conjugated antibodies specific for either of the recombinant proteins are then added to the cells and incubated for further 30 min followed by washing and fixing with 4% paraformaldehyde solution. Binding is then detected by FACS. Specificity is confirmed by adding a competing soluble synthetic peptide.

### In vitro analysis of the functional activity of TFP

TFPs function is confirmed using cytokine release assay with the transduced cells as effectors and target cell lines expressing any or all of the target antigens. HUVECS is used as target for the vasculature targeting as well as tumor cell lines expressing αvβ3 such as MDAMB435 breast carcinoma. To evaluate HER-2 specificity of the TFP, C1R/A2 and its transfectant C1R/A2HER2, which expresses HER-2 are used as well as carcinoma cell lines expressing HER-2. Functional specificity is confirmed by adding a competing soluble synthetic peptide. TFPs specificity for the gp33 and gp100₂₀₉ epitopes are confirmed by using peptide-pulsed RMA-S and T2 cells, respectively, and by using MC57-neo and MC57-gp33 expressing tumor cell lines and 624-28 (HLA-A2 negative and gp100 positive) and 624-38 (HLA-A2 and gp100 positive) melanoma cell lines.

### Adoptive therapy with TFP-engineered T cells targeting antigen-loss variants

The rational for this study is to answer the question of whether it is possible to provide an immunotherapeutic strategy that prevents "immune escape" of tumor cells expressing low amount of a targeted antigen. An MHC-restricted epitope, a cell surface-expressed proto-oncogene and tumor vasculature are targeted simultaneously.

MC57G tumor cell lines are used to compare the therapeutic potential of P14 TCR versus the TFPs based on P14 with the added specificity to HER-2 and neo-vasculature. Six different populations of T cells are used in the adoptive transfer:
1. OT-I
2. P 14 T cells from P14 TCR transgenic mice
3. P 14 transduced OT-I
4. P14 TFP transduced OT-I with specificities to H2Kb-gp33 and HER-2 (TFPh)
5. P14 TFP transduced OT-I with specificities to H2Kb-gp33 and angiogenesis (TFPv)
6. P14 TFP transduced OT-I with specificities to H2Kb-gp33, HER-2 and angiogenesis (TFPhv)

Rag-/- OT-I mice are challenged subcutaneously on contra-lateral sites with either of the following 4 tumor pairs:
A. MC57-neo and MC57-gp33_{low}
B. MC57-neo and MC57G-neo-HER-2
C. MC57G-neo-HER-2 and MC57G-gp33_{low}-HER-2
D. MC57G-neo and MC57G-gp33_{high}

Once the tumors reach 1 cm mean tumor diameter, each of the 4 groups receive intravenous injection with 10e6 activated CD8+ positive P14, OT-I, P14 transduced OT-I, TFP transduced OT-I T cells or TFP transduced OT-I T cells and the RGD4C synthetic peptide, which are used to block the recognition of the RGD4C sequence of the TFP. Efficient tumor destruction would require targeting tumor stroma either directly through RGD4C specificity of the TFP or through targeting stroma cells cross presenting the gp33 epitope. The expected recognition spectrum of these different effectors is outlined in table 2.

**Table 1. Amino acid sequences of peptides used in the TFPs.**

| **Peptide** | **Target** | **Sequence** | **Length aa** |
|---|---|---|---|
| myc-tag | myc antibody | EQKLISEEDL (SEQ ID NO:3) | 10 |
| 2xmyc-tag | myc antibody | EQKLISEEDLEQKLISEEDL (SEQ ID NO:4) | 20 |
| MARS | HER-2 | MARSGL (SEQ ID NO:1) | 6 |
| RGD4C | αv Integrins | CDCRGDCFC (SEQ ID NO:2) | 9 |

**Table 2. The predicted recognition pattern of transduced/transferred T cells.**

| | **Transferred T cells and recognition** | | | | | | |
|---|---|---|---|---|---|---|---|
| **Target** | OT-I | P14 | P14-OT-I | TFPh-OT-I | TFPv-OT-I | TFPhv-OT-I | TFPhv-OT-I +RGD4C peptide |
| H2Kb-gp33 | No | Yes | Yes | Yes | Yes | Yes | Yes |
| HER-2 | No | No | No | Yes | No | Yes | Yes |
| Neo-vasculature | No | No | No | No | Yes | Yes | No |

### Publications:

Arap, W., R. Pasqualini and E. Ruoslahti (1998). "Cancer treatment by targeted drug delivery to tumor vasculature in a mouse model." Science 279(5349): 377-80.
Folkman, J. (2007). "Angiogenesis: an organizing principle for drug discovery?" Nat Rev Drug Discov 6(4): 273-86.
Janeway, C. A., P. Travers, M. Walport and J. D. Capra (2004). Immunobiology, the immune system in health and disease. New York, Elsvevier Science Ltd/Garland Publishing.
Khong, H. T. and N. P. Restifo (2002). "Natural selection of tumor variants in the generation of "tumor escape" phenotypes." Nat Immunol 3(11): 999-1005.
Leen, A. M., C. M. Rooney and A. E. Foster (2007). "Improving T cell therapy for cancer." Annu Rev Immunol 25: 243-65.
Ljunggren, H. G. and K. J. Malmberg (2007). "Prospects for the use ofNK cells in immunotherapy of human cancer." Nat Rev Immunol 7(5): 329-39.
Matsui, K., L. A. O'Mara and P. M. Allen (2003). "Successful elimination of large established tumors and avoidance of antigen-loss variants by aggressive adoptive T cell immunotherapy." Int Immunol 15(7): 797-805.
McKee, M. D., A. Fichera and M. I. Nishimura (2007). "T cell immunotherapy." Front Biosci 12: 919-32.
Restifo, N. P., F. M. Marincola, Y. Kawakami, J. Taubenberger, J. R. Yannelli and S. A. Rosenberg (1996). "Loss of functional beta 2-microglobulin in metastatic melanomas from five patients receiving immunotherapy." J Natl Cancer Inst 88(2): 100-8.
Samoylova, T. I., N. E. Morrison, L. P. Globa and N. R. Cox (2006). "Peptide phage display: opportunities for development of personalized anti-cancer strategies." Anticancer Agents Med Chem 6(1): 9-17.
Schreiber, H., T. H. Wu, J. Nachman and W. M. Kast (2002). "Immunodominance and tumor escape." Semin Cancer Biol 12(1): 25-31.
Scott, J. K. (2001). Peptide Libraries. Phage Display: A Laboratory Manual. C. F. I. Barbas, D. R. Burton, J. K. Scott and G. J. Silverman. New York, CSH: 4. 1- 4. 13.
Seliger, B. (2005). "Strategies of tumor immune evasion." BioDrugs 19(6): 347-54.
Spiotto, M. T. and H. Schreiber (2005). "Rapid destruction of the tumor microenvironment by CTLs recognizing cancer-specific antigens cross-presented by stromal cells." Cancer Immun 5: 8.
Spiotto, M. T., P. Yu, D. A. Rowley, M. I. Nishimura, S. C. Meredith, T. F. Gajewski, Y. X. Fu and H. Schreiber (2002). "Increasing tumor antigen expression overcomes "ignorance" to solid tumors via crosspresentation by bone marrow-derived stromal cells." Immunity 17(6): 737-47.
Waldmann, T. A. (2006). "Effective cancer therapy through immunomodulation." Annu Rev Med 57: 65-81.
Wang, X., H. Zhao, Q. Xu, W. Jin, C. Liu, H. Zhang, Z. Huang, X. Zhang, Y. Zhang, D. Xin, A. J. Simpson, L. J. Old, Y. Na, Y. Zhao and W. Chen (2006). "HPtaa database-potential target genes for clinical diagnosis and immunotherapy of human carcinoma." Nucleic Acids Res 34(Database issue): D607-12.
Becker, C., H. Pohla, B. Frankenberger, T. Schuler, M. Assenmacher, D. J. Schendel and T. Blankenstein (2001). "Adoptive tumor therapy with T lymphocytes enriched through an IFN-gamma capture assay." Nat Med 7(10): 1159-62.
Blankenstein, T. and Z. Qin (2003). "The role of IFN-gamma in tumor transplantation immunity and inhibition of chemical carcinogenesis." Curr Opin Immunol 15(2): 148-54.
Charo, J. and P. F. Robbins (2007). "Contrasting effects of FLIPL overexpression in human T cells on activation-induced cell death and cytokine production." J Leukoc Biol.
Charo, J., S. E. Finkelstein, N. Grewal, N. P. Restifo, P. F. Robbins and S. A. Rosenberg (2005). "Bcl-2 overexpression enhances tumor-specific T-cell survival." Cancer Res 65(5): 2001-8.
Charo, J., J. A. Lindencrona, L. M. Carlson, J. Hinkula and R. Kiessling (2004). "Protective efficacy of a DNA influenza virus vaccine is markedly increased by the coadministration of a Schiff base-forming drug." J Virol 78(20): 11321-6.
Charo, J., M. Sundback, K. Wasserman, A. M. Ciupitu, B. Mirzai, R. van der Zee and R. Kiessling (2002). "Marked enhancement of the antigen-specific immune response by combining plasmid DNA-based immunization with a Schiff base-forming drug." Infect Immun 70(12): 6652-7.
Charo, J., A. Geluk, M. Sundback, B. Mirzai, A. D. Diehl, K. J. Malmberg, A. Achour, S. Huriguchi, K. E. van Meijgaarden, J. W. Drijfhout, N. Beekman, P. van Veelen, F. Ossendorp, T. H. Ottenhoff and R. Kiessling (2001). "The identification of a common pathogen-specific HLA class I A*0201-restricted cytotoxic T cell epitope encoded within the heat shock protein 65." Eur J Immunol 31(12): 3602-11.
Charo, J., M. Sundback, A. Geluk, T. Ottenhoff and R. Kiessling (2001). "DNA immunization of HLA transgenic mice with a plasmid expressing mycobacterial heat shock protein 65 results in HLA class I- and II-restricted T cell responses that can be augmented by cytokines." Hum Gene Ther 12(14): 1797-804.
Charo, J., A. M. Ciupitu, A. Le Chevalier De Preville, P. Trivedi, G. Klein, J. Hinkula and R. Kiessling (1999). "A long-term memory obtained by genetic immunization results in full protection from a mammary adenocarcinoma expressing an EBV gene." J Immunol 163(11): 5913-9.
Choudhury, A., J. Charo, S. K. Parapuram, R. C. Hunt, D. M. Hunt, B. Seliger and R. Kiessling (2004). "Small interfering RNA (siRNA) inhibits the expression of the Her2/neu gene, upregulates HLA class I and induces apoptosis of Her2/neu positive tumor cell lines." Int J Cancer 108(1): 71-7.
Gladow, M., W. Uckert and T. Blankenstein (2004). "Dual T cell receptor T cells with two defined specificities mediate tumor suppression via both receptors." Eur J Immunol 34(7): 1882-91.
Kono, K., Y. Rongcun, J. Charo, F. Ichihara, E. Celis, A. Sette, E. Appella, T. Sekikawa, Y. Matsumoto and R. Kiessling (1998). "Identification of HER2/neu-derived peptide epitopes recognized by gastric cancer-specific cytotoxic T lymphocytes." Int J Cancer 78(2): 202-8.
Kieback E, Charo J, Sommermeyer D, Blankenstein T, Uckert W (2008). A safeguard eliminates T cell receptor gene-modified autoreactive T cells after adoptive transfer. Proc Natl Acad Sci USA. 105(2):623-8.
Kruschinski A, Moosmann A, Poschke I, Norell H, Chmielewski M, Seliger B, Kiessling R, Blankenstein T, Abken H and J Charo (2008).Engineering antigen-specific primary human NK cells against HER-2 positive carcinomas. Proc Natl Acad Sci USA. 105(45):17481-6.
Leifert, J. A., J. A. Lindencrona, J. Charo and J. L. Whitton (2001). "Enhancing T cell activation and antiviral protection by introducing the HIV-1 protein transduction domain into a DNA vaccine." Hum Gene Ther 12(15): 1881-92.
Petersson, M., J. Charo, F. Salazar-Onfray, G. Noffz, M. Mohaupt, Z. Qin, G. Klein, T. Blankenstein and R. Kiessling (1998). "Constitutive IL-10 production accounts for the high NK sensitivity, low MHC class I expression, and poor transporter associated with antigen processing (TAP)-1/2 function in the prototype NK target YAC-1." J Immunol 161(5): 2099-105.
Qin, Z. and T. Blankenstein (2004). "A cancer immunosurveillance controversy." Nat Immunol 5(1): 3-4; author reply 4-5.
Qin, Z., J. Schwartzkopff, F. Pradera, T. Kammertoens, B. Seliger, H. Pircher and T. Blankenstein (2003). "A critical requirement of interferon gamma-mediated angiostasis for tumor rejection by CD8+ T cells." Cancer Res 63(14): 4095-100.
Qin, Z., H. J. Kim, J. Hemme and T. Blankenstein (2002). "Inhibition of methylcholanthrene-induced carcinogenesis by an interferon gamma receptor-dependent foreign body reaction." J Exp Med 195(11): 1479-90.
Qin, Z. and T. Blankenstein (2000). "CD4+ T cell--mediated tumor rejection involves inhibition of angiogenesis that is dependent on IFN gamma receptor expression by nonhematopoietic cells." Immunity 12(6): 677-86.
Rongcun, Y., F. Salazar-Onfray, J. Charo, K. J. Malmberg, K. Evrin, H. Maes, K. Kono, C. Hising, M. Petersson, O. Larsson, L. Lan, E. Appella, A. Sette, E. Celis and R. Kiessling (1999). "Identification of new HER2/neu-derived peptide epitopes that can elicit specific CTL against autologous and allogeneic carcinomas and melanomas." J Immunol 163(2): 1037-44.
Roos, A. K., M. Pavlenko, J. Charo, L. Egevad and P. Pisa (2005). "Induction of PSA-specific CTLs and anti-tumor immunity by a genetic prostate cancer vaccine." Prostate 62(3): 217-23.
Salazar-Onfray, F., J. Charo, M. Petersson, S. Freland, G. Noffz, Z. Qin, T. Blankenstein, H. G. Ljunggren and R. Kiessling (1997). "Down-regulation of the expression and function of the transporter associated with antigen processing in murine tumor cell lines expressing IL-10." J Immunol 159(7): 3195-202.
Schuler, T. and T. Blankenstein (2003). "Cutting edge: CD8+ effector T cells reject tumors by direct antigen recognition but indirect action on host cells." J Immunol 170(9): 4427-31.
Schuler, T., T. Kammertoens, S. Preiss, P. Debs, N. Noben-Trauth and T. Blankenstein (2001). "Generation of tumor-associated cytotoxic T lymphocytes requires interleukin 4 from CD8(+) T cells." J Exp Med 194(12): 1767-75.
Sommermeyer, D., J. Neudorfer, M. Weinhold, M. Leisegang, B. Engels, E. Noessner, M. H. Heemskerk, J. Charo, D. J. Schendel, T. Blankenstein, H. Bernhard and W. Uckert (2006). "Designer T cells by T cell receptor replacement." Eur J Immunol 36(11): 3052-9.
Willimsky, G. and T. Blankenstein (2000). "Interleukin-7/B7.1-encoding adenoviruses induce rejection of transplanted but not nontransplanted tumors." Cancer Res 60(3): 685-92.
Wilson, J. L., J. Charo, A. Martin-Fontecha, P. Dellabona, G. Casorati, B. J. Chambers, R. Kiessling, M. T. Bejarano and H. G. Ljunggren (1999). "NK cell triggering by the human costimulatory molecules CD80 and CD86." J Immunol 163(8): 4207-12.
Wilson, J. L., L. C. Heffler, J. Charo, A. Scheynius, M. T. Bejarano and H. G. Ljunggren (1999). "Targeting of human dendritic cells by autologous NK cells." J Immunol 163(12): 6365-70.

## Claims

1. A functional TCR α and/or β chain fusion protein recognizing and binding to at least one MHC-presented epitope, and containing at least one amino acid sequence recognizing and binding an antigen.

2. The TCR fusion protein of claim 1, wherein the epitope is a tumor cell epitope and the antigen is a tumor cell antigen.

3. The TCR fusion protein of claim 2, wherein the antigen is a cell-surface antigen, a further MHC-presented epitope or an extracellular antigen, preferably a neo-vasculature specific antigen.

4. The TCR fusion protein of claims 2 and 3, wherein the cell surface antigen or the further MHC-presented epitope is derived from HER2, CEA, PSMA, CD20.

5. The TCR fusion protein of one or more of the preceding claims, wherein the epitope is selected from gp100 or gp1, or wherein the neo-vasculature specific antigen is integrin αvβ3 or αvβ5.

6. The TCR fusion protein of one or more of the preceding claims, wherein
i) the one or more antigen binding amino acid sequences are added to the N-terminus of the α and/or β chain,
ii) the one or more antigen binding amino acid sequences are inserted into a constant or a variable region of said α and/or β chain, and/or
iii) the one or more antigen binding amino acid sequences are replacing a number of amino acids in a constant or a variable region of said α and/or β chain.

7. The TCR fusion protein of claim 6, wherein said fusion protein comprises the one or more antigen binding amino acid sequences either spaced apart or directly in tandem, or wherein the TCR is expressed as a single chain or soluble receptor.

8. The TCR α and/or β chain fusion protein of one or more of the preceding claims, further comprising at least one epitope-tag, wherein said epitope-tag is selected from
i) an epitope-tag added to the N- and/or C-terminus of the α and/or β chain,
ii) an epitope-tag inserted into a constant or variable region of said α and/or β chain, and
iii) an epitope-tag replacing a number of amino acids in a constant or variable region of said α and/or β chain.

9. The TCR fusion protein of one or more of the preceding claims, wherein the amino acid sequence recognizing and binding an antigen has a length of between 5-20 amino acids, preferably between 6 and 12 amino acids, and wherein the amino acid sequence recognizing and binding an antigen preferably is selected from SEQ ID NO: 1 and/or 2.

10. An isolated nucleic acid molecule coding for the TCR fusion protein of one or more of the preceding claims.

11. The nucleic acid molecule according to claim 10, wherein said molecule is selected from DNA, RNA, PNA, CNA, mRNA or mixtures thereof.

12. A vector, preferably a plasmid, shuttle vector, phagemide, cosmid, expression vector, retroviral vector, adenoviral vector or particle and/or vector to be used in gene therapy, which comprises a nucleic acid molecule of claims 10 or 11.

13. A host cell, transfected with a vector of claim 12 or a nucleic acid of claim 10, or infected or transduced with a particle according to claim 12, wherein said cell preferably is a T-cell, a natural killer cell, a monocyte, a natural killer T-cell, precursor cell, a hematopoietic stem cell or a non-pluripotent stem cell.

14. The host cell according to claim 13, wherein said host cell expresses a fusion protein according to any of claims 1 to 9 on its surface.

15. A method for producing a fusion protein according to any of claims 1 to 9, comprising a chemical synthesis or genetic engineering of said peptide, or
comprising expressing a nucleic acid molecule in a host cell according to claim 13 or 14, and purifying said fusion protein or said TCR from said host cell.

16. A pharmaceutical composition, comprising a fusion protein according to any of claims 1 to 9, a nucleic acid molecule according to claim 10 or 11, a vector according to claim 12 or a host cell according to any of claims 13 to 14, together with a pharmaceutically acceptable carrier or excipient.

17. Use of a pharmaceutical composition according to claim 16 for the manufacture of a medicament for the treatment of tumor, autoimmune, hereditary, or infectious diseases, immunodeficiency, allergy and/or for use in transplantation.
